# EUROPEAN PATENT SPECIFICATION

(11) **EP 1 817 325 B1**
(45) Date of publication and mention of the grant of the patent: **21.01.2009**
(21) Application number: 05800868.1
(22) Date of filing: 20.09.2005
(51) Int. Cl.: C07J 41/00

(54) **SULFATED MOLECULAR UMBRELLAS CONTAINING 6-200 CHOLANIC ACID (DERIVATIVE) UNITS AS ANTI-HIV AND ANTI-HSV AGENTS**
6-200 CHOLANSÄURE(DERIVAT)-EINHEITEN ENTHALTENDE SULFATIERTE MOLEKULARE SCHIRME ALS ANTI-HIV- UND ANTI-HSV-MITTEL
PARAPLUIES MOLECULAIRES SULFATES CONTENANT 6-200 ACIDES CHOLARIQUES (DERIVES) EN TANT QU' AGENTS ANTI-VIH ET ANTI-VHS

(30) Priority: 21.09.2004 US 611306 P
(43) Date of publication of application: 15.08.2007
(73) Proprietor: Lehigh University, Bethlehem, PA 18215 (US); The Mount Sinai School of Medicine of New York University, New York, NY 10029-6547 (US)
(72) Inventor: REGEN, Steven, Allentown, PA 18104 (US); HEROLD, Betsy, C., Scarsdale, NY 10583 (US)
(74) Representative: Wallace, Sheila Jane
(86) International application number: PCT/US2005/033880
(87) International publication number: WO 2006/034369

(56) References cited:
- US-B1- 6 190 650
- JING, JANOUT, HEROLD, KLOTMAN HEALD, REGEN: "Persulfated molecular umbrellas as anti-HIV and anti-HSV agents" JOURNAL OF THE AMERICAN CHEMICAL SOCIETY, vol. 126, 18 November 2004 (2004-11-18), pages 15930-15931, XP002369337
- BANDYOPADHYAY, JANOUT, ZHANG, REGEN: "Ion conductors derived from cholic acid and spermine: importance of facial hydrophilicity on Na+ transport and membrane selectivity" JOURNAL OF THE AMERICAN CHEMICAL SOCIETY, vol. 123, 2001, pages 7691-7696, XP002369338
- JANOUT, STAINA, BANDYOPADHYAY, REGEN: "Evidence of an umbrella mechanism of bilayer transport" JOURNAL OF THE AMERICAN CHEMICAL SOCIETY, vol. 123, 2001, pages 9926-9927, XP002369339
- REISING, BOURNE, EL-AWAR, STANBERRY: "Selective activity of cholic acid and related compounds against herpes simplex virus type 2." 9TH INTERNATIONAL CONFERENCE ON ANTIVIRAL RESEARCH. ANTIVIRAL RESEARCH, 1996, page A46, XP002369343

## Description

### Field of the Invention

The invention relates to a chemical genus of sulfated cholestanes attached to an oligomer through amide or ester bonds. The compounds are useful as anti human immunodeficiency virus (HIV) and anti-herpes simplex virus (HSV) agents.

### Background of the Invention

Molecular umbrella molecules interconvert between two different morphological states in response to changes in microenvironment. Such "amphomorphic" compounds mimic, in a sense, the structure and function of umbrellas. Thus, when two or more facially amphiphilic units (i.e., rigid hydrocarbon units that maintain a hydrophobic and a hydrophilic face) are coupled to a suitable scaffold, immersion in an aqueous environment favors an exposed (or fully-exposed) conformation, where hydrophobic interactions are maximized and where the external face of each "wall" is hydrated. When immersed in a hydrocarbon environment (e.g., the interior of a lipid bilayer), the umbrella then favors a shielded conformation such that intramolecular dipole-dipole and hydrogen bonding interactions are maximized, and each hydrophobic face is effectively solvated by the hydrophobic microenvironment. Lipid bilayers play a central role in cells by serving as selective barriers for transport. In particular, they permit the passage of those molecules and ions that are necessary for maintaining the living state. At the same time, their hydrophobic core restricts the entry of many classes of biologically-active agents. Although the inventor does not wish to be bound to this hypothesis, it is possible that molecular umbrella conjugates can cross lipid bilayers through the following sequence of events: (i) diffusion to the membrane surface in an exposed or fully exposed state, (ii) insertion into the outer monolayer leaflet by flipping into a shielded state, (iii) diffusion to the inner monolayer leaflet, and (iv) entry into the adjoining aqueous phase via the reversal of steps ii and i. Recent kinetic studies have provided support for such a mechanism. In the present invention, persulfated molecular umbrellas might exert their anti-viral activity by inhibiting cellular binding of enveloped viruses by competing for specific glycoproteins on the viral envelope.

Unlike conventional polyanions, persulfated molecular umbrellas are expected to cross hydrophobic barriers; e.g., the blood-brain barrier. Thus, they have greater potential for systemic use and equal potential for topical applications relative to conventional polyanions. They offer greater versatility as antiviral agents. Second, the size, flexibility, and also the number and spatial distribution of the pendant sulfate groups of persulfated molecular umbrellas are readily optimized. Third, the fact that persulfated molecular umbrellas are derived from biogenic starting materials make them potentially biodegradable. Fourth, the synthesis of persulfated molecular umbrellas is straightforward and is based on inexpensive starting materials.
US-B1-6 190 650 discloses sulfonated dendrimers. This sulfonated groups are linked to an aromatic moiety and not to a cholanic acid derivative.
BANDYOPADHYAY, JANOUT, ZHANG, REGEN: "Ion conductors derived from cholic acid and spermine: importance of facial hydrophilicity on Na+ transport and membrane selectivity" JOURNAL OF THE AMERICAN CHEMICAL SOCIETY, vol. 123, 2001, pages 7691-7696 discloses compounds comprising 4 cholanic acid units. These have antifungal and antibacterial properties and are characterized by their ion-conducting properties. On the binding paragraph between the two columns of p. 7696, the ability of this ion conductors to differentiate between mammalian and microorganism membranes is mentioned. No reference to viruses is made, but the skilled person will deduce from this paragraph that encapsulated viruses would also fall within the target microorganisms.
JANOUT, STAINA, BANDYOPADHYAY, REGEN: "Evidence of an umbrella mechanism of bilayer transport" JOURNAL OF THE AMERICAN CHEMICAL SOCIETY, vol. 123, 2001, pages 9926-9927 discloses a compound comprising two cholanic acid moieties non sulfated. This compound is used as a transporter for gluthatione across membranes.
REISING, BOURNE, EL-AWAR, STANBERRY: "Selective activity of cholic acid and related compounds against herpes simplex virus type 2." 9TH INTERNATIONAL CONFERENCE ON ANTIVIRAL RESEARCH. ANTIVIRAL RESEARCH, 1996, page A46 discloses the therapeutical use of some cholanic acid derivatives against herpes virus. None of the derivatives contain two or more sulfate groups attached to the cholanic acid.

### Brief Summary of the Invention

In one aspect, the invention relates to compounds of formula 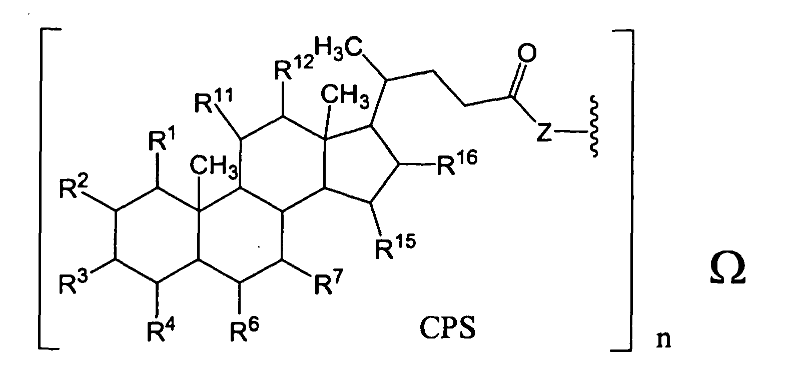 in which CPS (the structure within the square brackets) is a cholanic acid derivative. R¹ to R¹⁶ are chosen independently from H, OH and OSO₃⁻A⁺. W is a direct bond or a deshydrogen residue of a C3-C6 diol or triol. A is chosen from H⁺, a quaternary ammonium species and the cation of an alkali or alkaline earth metal. Z is a direct bond or a residue of an amino acid, a peptide or an aminosulfonic acid. Ω is a moiety having n or more -NH and -OH groups separated by 6 or fewer carbons; and
n is an integer from 6 to 200. Because cholanes have an α face and a β face, at least two of R¹ to R¹⁶ should be -W-OSO₃⁻A⁺ and should be on the same face of CPS so that the molecule can function as an umbrella.

In a second aspect, the invention relates to the use of the compounds of the invention for the manufacture of a medicament for inhibiting the infectivity of a virus.

In a third aspect, the invention relates to a pharmaceutical composition comprising a pharmaceutically acceptable carrier and a compound according to the invention.

### Detailed Description of the Invention

The invention relates to compounds of formula 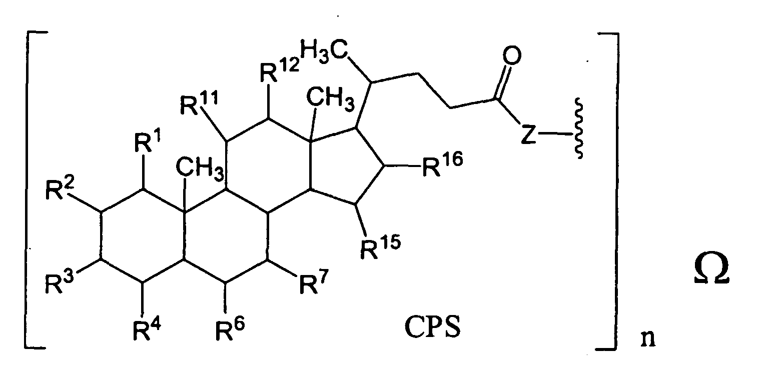 in which CPS is a cholanic acid derivative. CPS is a polysulfate of cholanic acid in which at least two hydroxyls of a parent cholanic acid are sulfated. The sulfate may be in the form of the free acid or an appropriate salt. The cholanic acid derivative is attached to Ω through an amide or ester bond formed between the carboxylic acid residue of the cholanic acid and an amine or hydroxyl of the Ω.

The compounds of the invention are aggregate molecules comprising (1) a plurality of rigid scaffolds, each optimally roughly planar, having two faces, one of which is hydrophobic and one of which is hydrophilic; and (2) a backbone that allows the hydrophobic and hydrophilic faces to exchange places on the surface of the aggregate molecule.

The backbone has been designated Ω. It arises from a compound which, before reacting with CPS, has at least 6 -NH and -OH groups separated by 6 or fewer carbons. Preferably there are from 8 to 24 -NH and/or -OH groups to which the cholanic acid derivative is attached. As is evident to person of skill in the art, when the CPS is attached, the -NH- and -OH groups give up their hydrogens and become-NH-, -N- and -O- linkages. Ω moieties exhibiting the desired properties include dendritic oligomers and linear oligomers. Examples include poly(lysine), poly(vinyl alcohol) and poly (ethyleneimine). In one embodiment, Ω is a spermine/lysine backbone. An example of such a backbone is 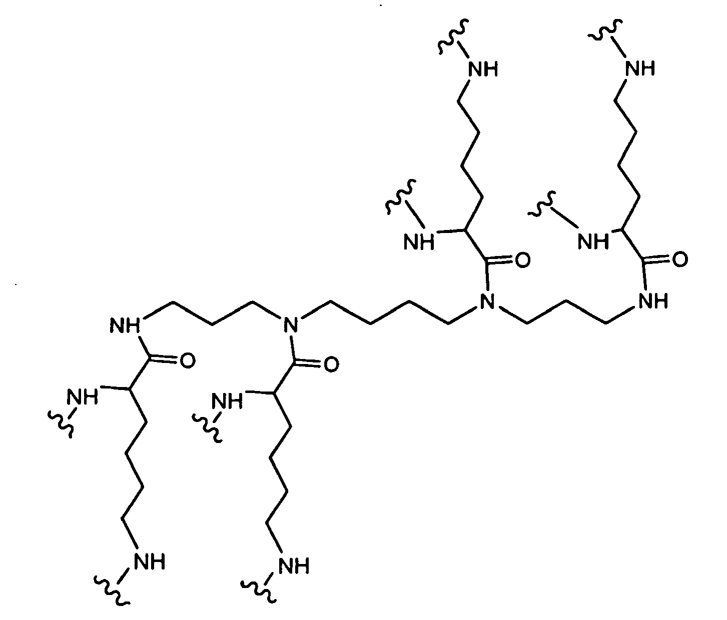 wherein the wavy lines indicate the points of attachment of CPS residues.

This backbone is derived from 4 units of lysine attached to spermine via amide bonds between the carboxyl of the lysine and each of the nitrogens of spermine. We refer to it as a spermine/lysine₄ backbone. Another arrangement of a spermine/lysine₄ backbone is 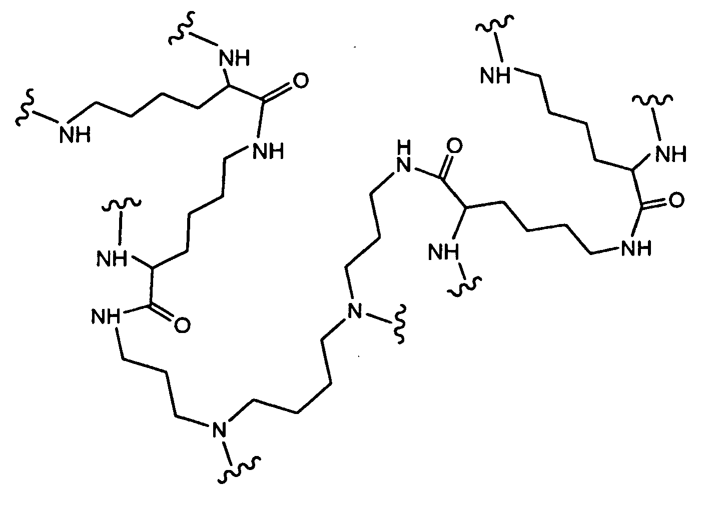 Both of these backbones allow the incorporation of 8 sterols (CPS elements).

In the compounds of the invention, the function of the rigid scaffold is provided by a cholanic acid derivative. Cholanic acid is

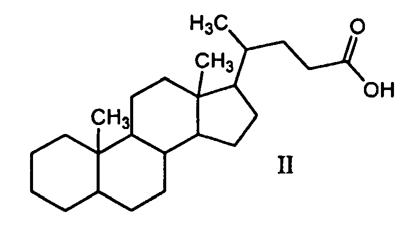

The parent term "cholanic acid" typically refers to a specific isomer IIa:

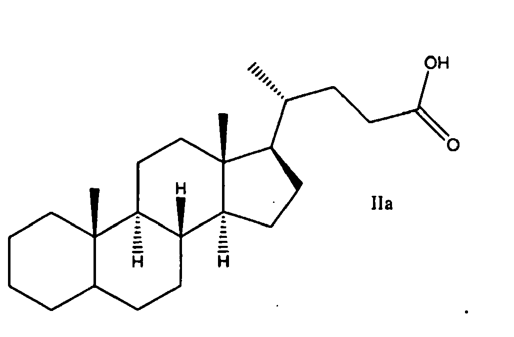

Sterols having the stereochemistry shown in IIa are preferred because they are readily commercially available, but the term "cholanic acid" as used herein refers to any isomer of formula II, since it will be evident to the artisan that any isomer having two faces would function for the intended purpose. Readily available cholanic acid derivatives include 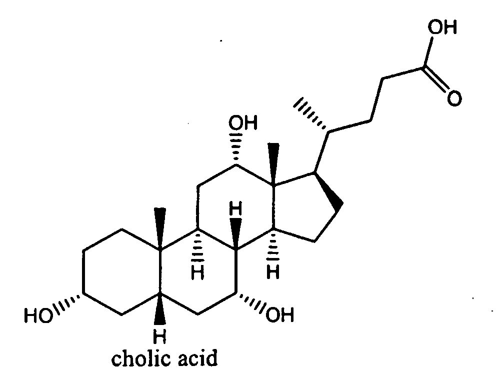 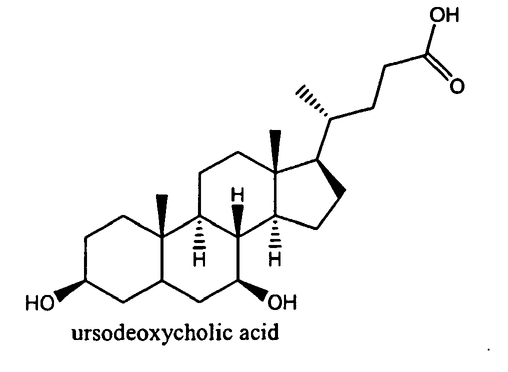 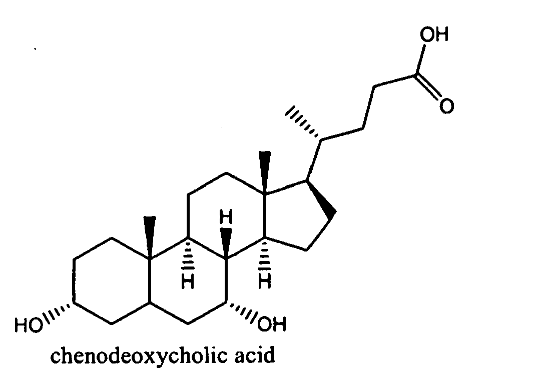 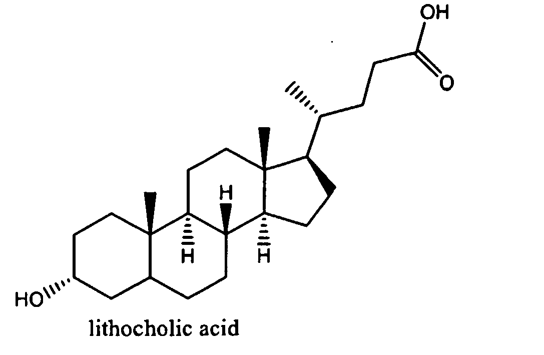 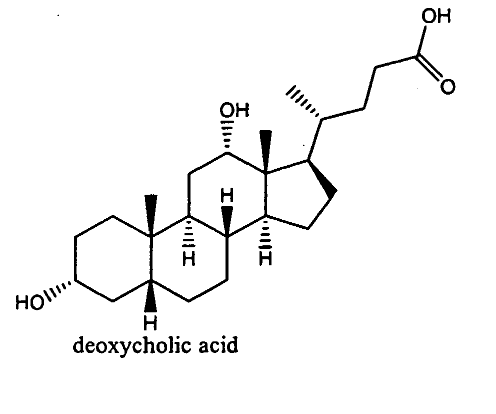 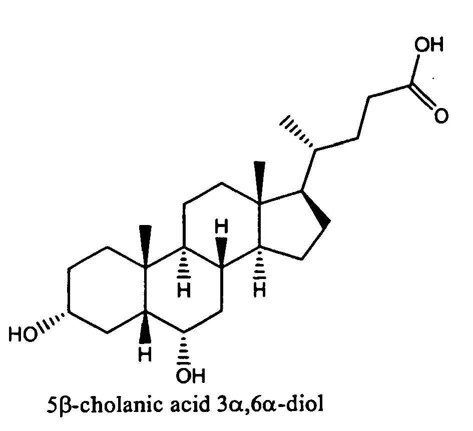 and many other similar sterols carrying various numbers and locations of hydroxyls.

Other possibilities for scaffolds, in which Z is not a direct bond, include glycocholic acid, taurocholic acid and their derivatives:

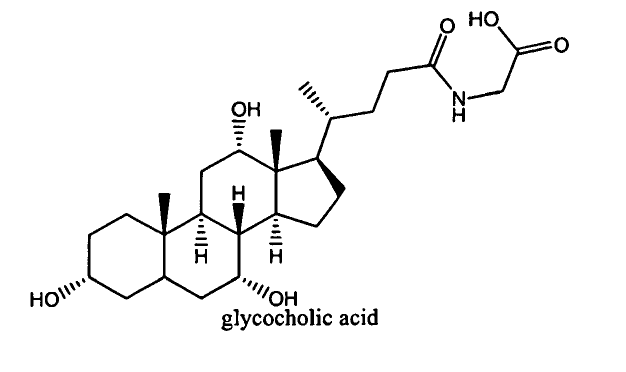

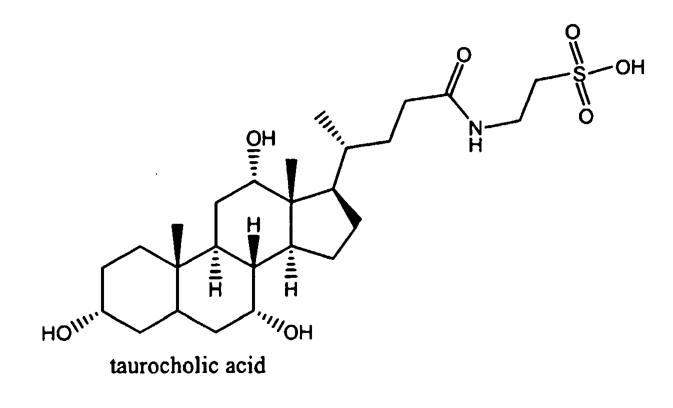

Taurocholic acid derivatives would be attached to the backbone by a sulfonamide or sulfate bond rather than a carboxamide or ester, but the chemistries are all fundamentally similar and well known to persons of skill in the art.

These derivatives provide compounds in which Z is a residue of an amino acid (e.g. glycine in glycocholic acid), a peptide (not shown) or an aminosulfonic acid (e.g. taurine in taurocholic acid.) The individual hydroxyls on the cholanic acid derivative are sulfated, as described below, after the appropriate cholanic acid derivative has been attached to the backbone. The chemistries for forming amides from amines and carboxylic acids and esters from alcohols and acids are well known to the artisan. The overall synthesis of compounds of the invention is shown in Scheme 1 below for an example in which Ω is a spermine/lysine₄ backbone and the particular cholanic acid polysulfate (CPS) is cholic acid trisulfate (CTS). For convenience, we will use shorthand in which substitution on the lysine α-amine is unbracketed or is on the same line as the lysine residue; substitution on the lysine ε-amine is in parentheses or is above or below the line of the lysine residue. Cholic acid will be abbreviated as "Chol", and the number of such residues will be shown as Cholₙ. The terminology is illustrated below:

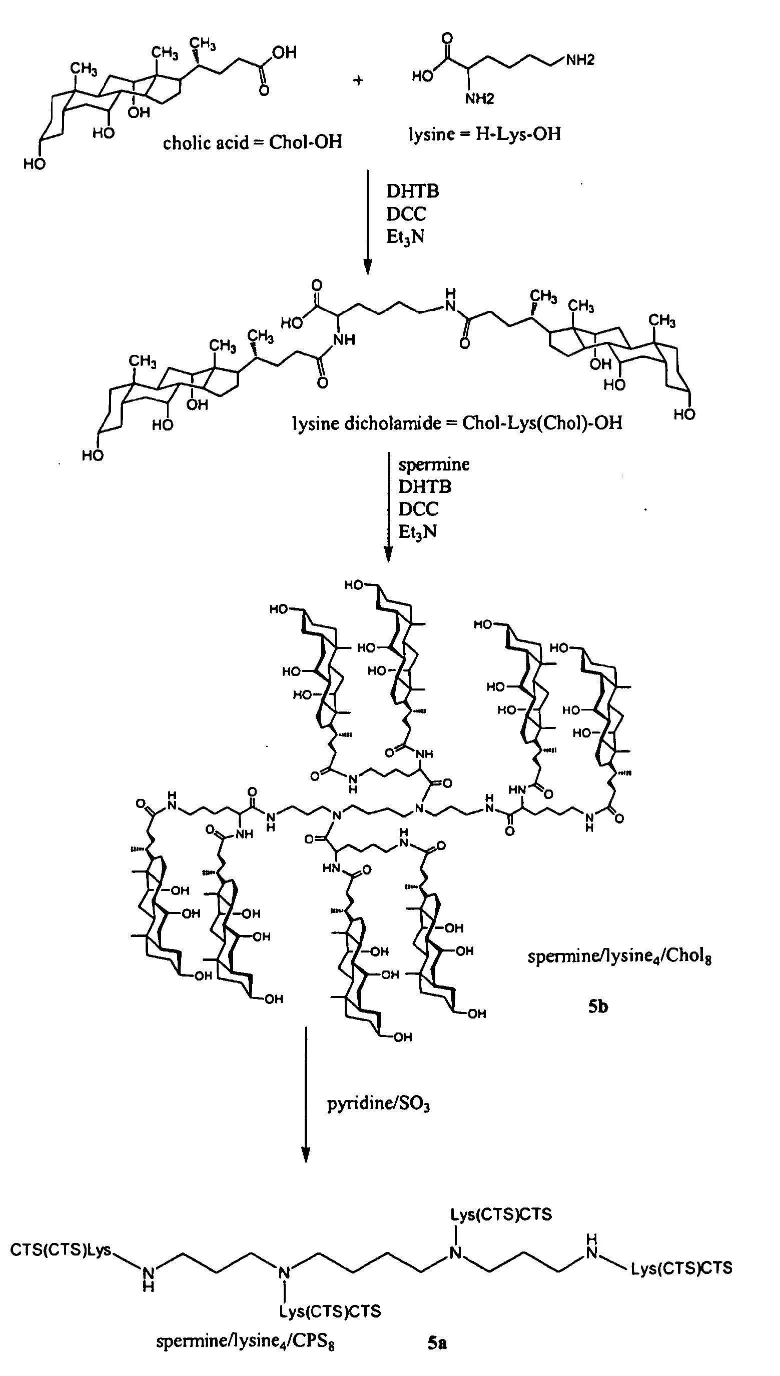

Other examples of compounds of the invention include: 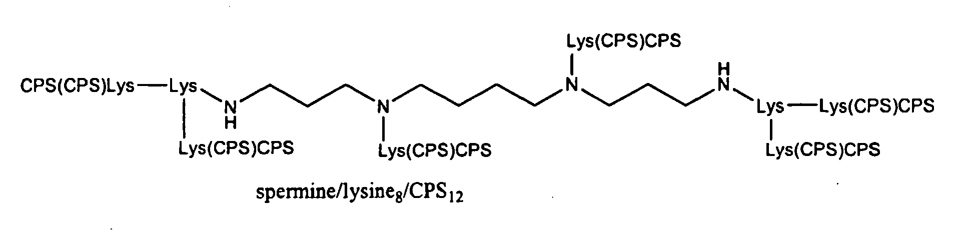 and similarly

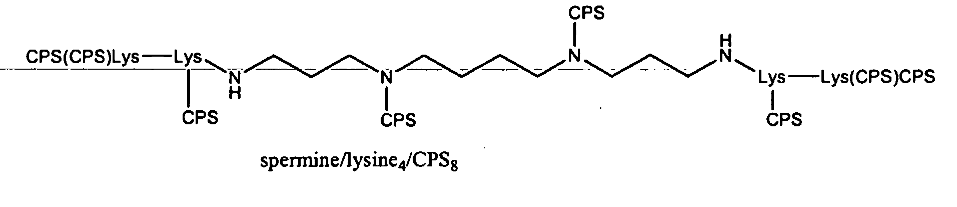

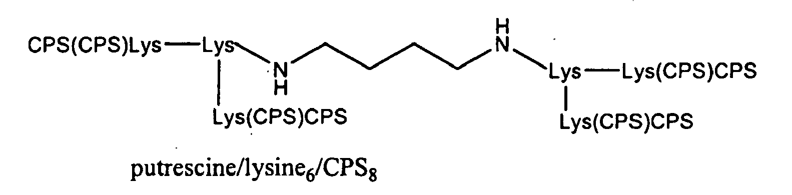

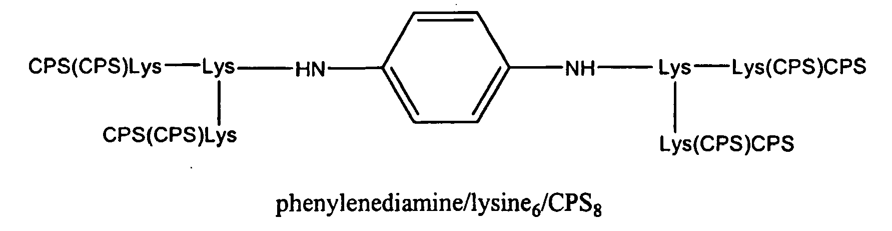

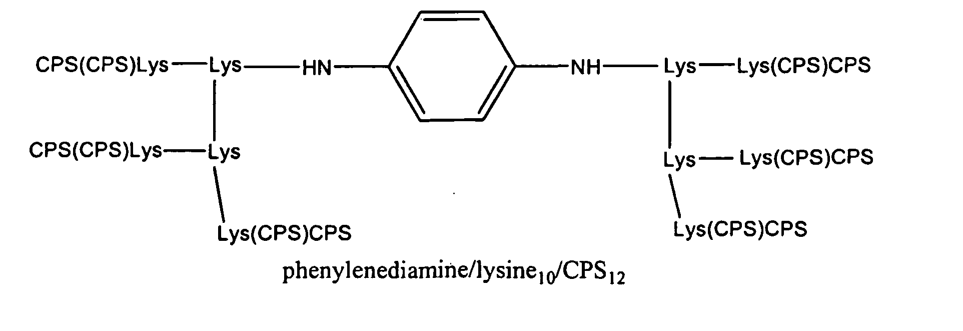

The synthesis of pheneylenediamine/lysine₁₀/CTS₁₂ (where CTS is cholic acid trisulfate) is shown schematically below. It illustrates the common steps and reagents for all such syntheses:

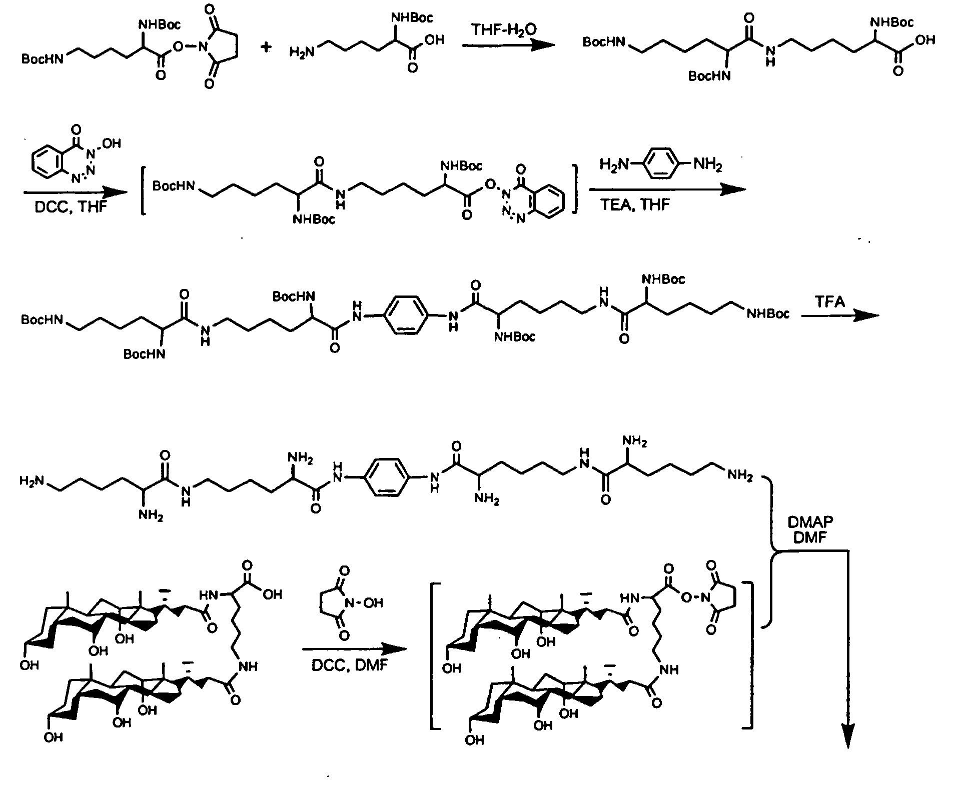

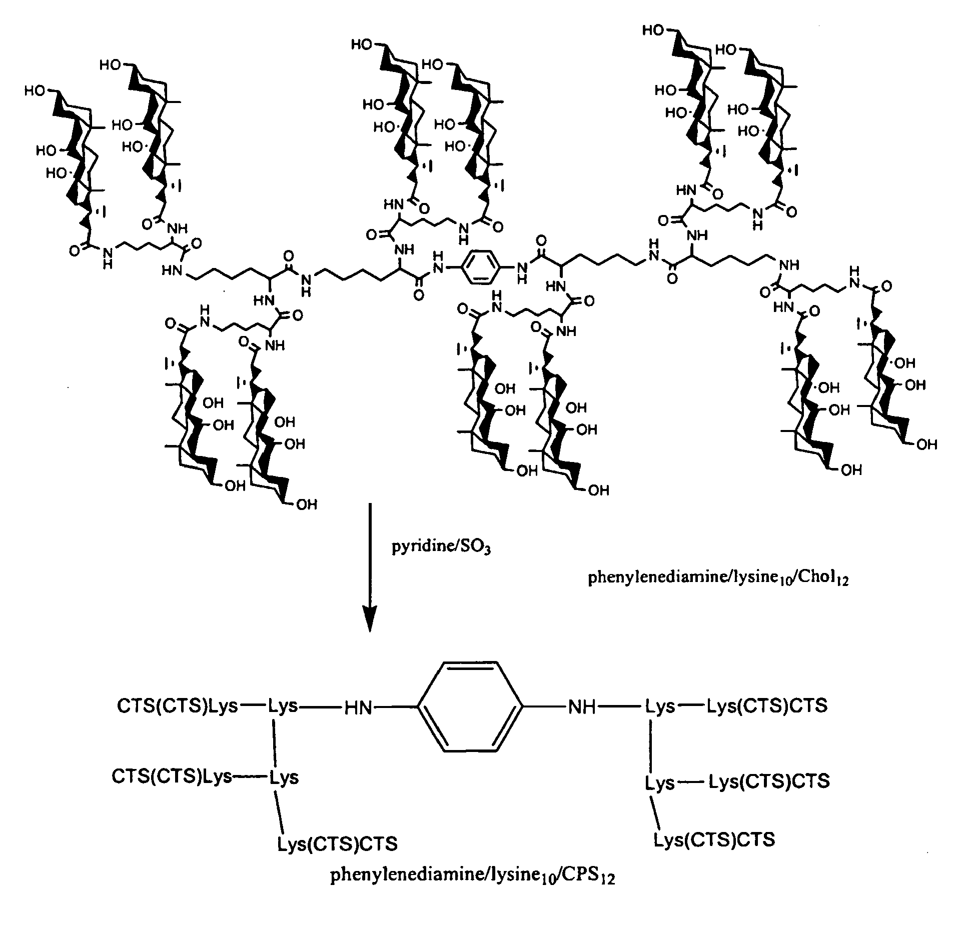

The number of sulfates in most embodiments is the same as the number of hydroxyls on the cholanic acid derivative. However, if more sulfates are desired, the number can be multiplied by first attaching an aminopolyol to one or more hydroxyls on the cholanic acid derivative. In that case, W becomes a deshydrogen residue of a polyol. Typical polyols are diethanolamine and tris(hydroxymethyl)aminomethane, which exemplify C₃-C₆ diols or triols. They may be attached by activating the C-3 hydroxyl group of cholic acid methyl ester with phosgene to afford the corresponding chloroformate quantitatively. Subsequent reaction with diethanolamine or tris(hydroxymethyl)aminomethane, followed by mild hydrolysis of the methyl ester, gives 9 and 10.

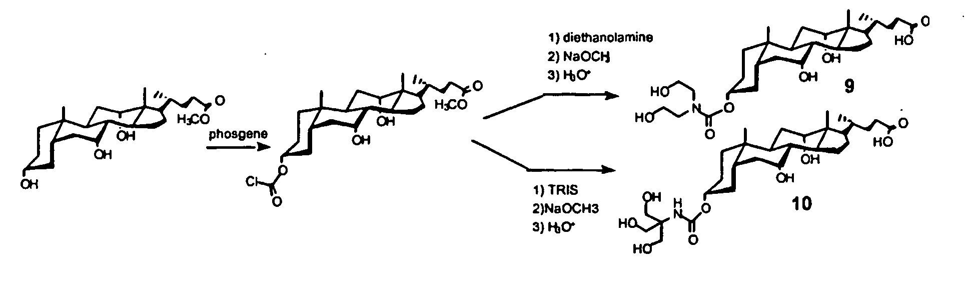

From these, one may prepare a variety of molecular umbrellas using synthetic procedures analogous to those below. Using spermine, lysine and cholic acid, one can create many arrangements that provide whatever number of sulfate groups is desired. The same variations can be realized by employing putrescine or spermidine in place of spermine; by using ornithine; 2,3-diaminopropionic acid; 2,4-diaminobutyric acid; 2-methylomithine, N-ε-methyl-L-lysine, in place of lysine and by using any of the readily available cholanic acid derivatives in place of cholic acid. Alternatively, one could employ serine or threonine as the amino acid, and attach the sterol via a mixture of ester and amide linkages. For most amino acids, the L enantiomer is more readily available, and will be preferred for that reason, but the invention is not restricted to L isomers. Similarly, amino acids are not restricted to α-amino acids, although except for β-alanine and taurine (which are contemplated for the residue "Z"), they are the most commonly available.

The compounds of the invention inhibit the infectivity of viruses. They may be employed either as topical antiviral agents or as systemic antiviral agents, depending on the therapeutic ratio, bioavailability and pharmacodynamics of a particular compound. Compositions of the invention comprise an effective dose or a pharmaceutically effective amount or a therapeutically effective amount of a compound described above and may additionally comprise one or more additional antiviral agents. Methods of the invention parallel the compositions and formulations. The methods comprise bringing a virus into contact with a therapeutically effective amount of a compound described above. This can be accomplished by administering to a patient in need of treatment a therapeutically effective amount of a compound according to the invention or by applying a therapeutically effective amount of a compound described above to the skin of a patient in need of treatment.

The compounds of the invention are sulfates. Therefore they will commonly be presented as salts. In the claims, reference to the acid includes its salts. The term "pharmaceutically acceptable salt" refers to salts whose counter ion derives from pharmaceutically acceptable non-toxic bases. Suitable pharmaceutically acceptable base addition salts for the compounds of the present invention include ammonium salts, metallic salts made from, for example, aluminum, calcium, iron, lithium, magnesium, manganese, potassium, sodium and zinc and organic salts made from, for example, lysine, N,N'-dibenzylethylenediamine, chloroprocaine, choline, diethanolamine, ethylenediamine, meglumine (N-methylglucamine) and procaine. Other base addition salts includes those made from: arecoline, arginine, benethamine, benzathine, betaine, clemizole, deanol, diethylamine, diethylaminoethanol, epolamine, ethylenediamine, glucamine, glucosamine, histidine, hydrabamine, imidazole, isopropylamine, methylglucamine, morpholine, morpholineethanol, n-ethylmorpholine, n-ethylpiperidine, piperazine, piperidine, polyamine resins, purines, theobromine, triethylamine, trimethylamine, tripropylamine, trolamine, and tromethamine.

### Definitions

Throughout this specification the terms and substituents retain their definitions.

Alkyl is intended to include linear, branched, or cyclic hydrocarbon structures and combinations thereof. When not otherwise restricted, the term refers to alkyl of 20 or fewer carbons. Lower alkyl refers to alkyl groups of 1, 2, 3, 4, 5 and 6 carbon atoms. Examples of lower alkyl groups include methyl, ethyl, propyl, isopropyl, butyl and s-and t-butyl, Methyl is preferred. Preferred alkyl and alkylene groups are those of C₂₀ or below. Cycloalkyl is a subset of alkyl and includes cyclic hydrocarbon groups of 3, 4, 5, 6, 7, and 8 carbon atoms. Examples of cycloalkyl groups include c-propyl; c-butyl, c-pentyl, norbomyl and adamantyl.

C₁ to C₂₀ Hydrocarbon includes alkyl, cycloalkyl, alkenyl, alkynyl, aryl and combinations thereof. Examples include benzyl, phenethyl, cyclohexylmethyl, camphoryl and naphthylethyl.

Alkoxy or alkoxyl refers to groups of 1, 2, 3, 4, 5, 6, 7 or 8 carbon atoms of a straight, branched, cyclic configuration and combinations thereof attached to the parent structure through an oxygen. Examples include methoxy, ethoxy, propoxy, isopropoxy, cyclopropyloxy and cyclohexyloxy. Lower-alkoxy refers to groups containing one to four carbons.

Polyol refers to a compound or residue having a plurality of -OH groups. Polyols may be thought of as alkyl derivatives - in this case alkylamine derivatives - in which a plurality of C-H bonds have been replaced by C-OH bonds.

The term "halogen" means fluorine, chlorine, bromine or iodine.

The term "a deshydrogen residue of a polyol" when used to describe "W", refers to a polyol (as defined above) minus the hydrogens of the alcohols and amine. For example, the deshydrogen residue of diethanolamine is: 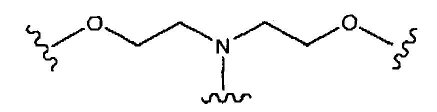 This is not *sensu stricto* a polyol, since it lacks two hydroxyls, which have become ethers. This and similar structures that lack hydrogens at the points of attachment are referred to herein as "deshydrogen residues of polyols".

Terminology related to "protecting", "deprotecting" and "protected" functionalities occurs throughout this application. Such terminology is well understood by persons of skill in the art and is used in the context of processes which involve sequential treatment with a series of reagents. In that context, a protecting group refers to a group which is used to mask a functionality during a process step in which it would otherwise react, but in which reaction is undesirable. The protecting group prevents reaction at that step, but may be subsequently removed to expose the original functionality. The removal or "deprotection" occurs after the completion of the reaction or reactions in which the functionality would interfere. Thus, when a sequence of reagents is specified, as it is in the processes of the invention, the person of ordinary skill can readily envision those groups that would be suitable as "protecting groups. Suitable groups for that purpose are discussed in standard textbooks in the field of chemistry, such as Protective Groups in Organic Synthesis by T.W.Greene [John Wiley & Sons, New York, 1991]. Particular attention is drawn to the chapters entitled "Protection for the Hydroxyl Group, Including 1,2- and 1,3-Diols" (pages 10-86).

The abbreviations Me, Et, Ph, Tf, Ts and Ms represent methyl, ethyl, phenyl, trifluoromethanesulfonyl, toluenesulfonyl and methanesulfonyl respectively. A comprehensive list of abbreviations utilized by organic chemists (i.e. persons of ordinary skill in the art) appears in the first issue of each volume of the Journal of Organic Chemistry, typically presented in a table entitled "Standard List of Abbreviations".

While it may be possible for the compounds of the invention to be administered as the raw chemical, it is preferable to present them as a pharmaceutical composition. According to a further aspect, the present invention provides a pharmaceutical composition comprising a compound of the invention or a pharmaceutically acceptable salt or solvate thereof, together with one or more pharmaceutical carriers thereof and optionally one or more other therapeutic ingredients. The carrier(s) must be "acceptable" in the sense of being compatible with the other ingredients of the formulation and not deleterious to the recipient thereof.

The formulations include those suitable for oral, parenteral (including subcutaneous, intradermal, intramuscular, intravenous and intraarticular), rectal and topical (including dermal, buccal, sublingual and intraocular) administration. The most suitable route may depend upon the condition and disorder of the recipient. The formulations may conveniently be presented in unit dosage form and may be prepared by any of the methods well known in the art of pharmacy. All methods include the step of bringing into association a compound of the invention or a pharmaceutically acceptable salt or solvate thereof ("active ingredient") with the carrier, which constitutes one or more accessory ingredients. In general, the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Molded tablets may be made by molding in a suitable machine a mixture of the powdered compound moistened with an inert liquid diluent. The tablets may optionally be coated or scored and may be formulated so as to provide sustained, delayed or controlled release of the active ingredient therein.

The pharmaceutical compositions may include a "pharmaceutically acceptable inert carrier", and this expression is intended to include one or more inert excipients, which include starches, polyols, granulating agents, microcrystalline cellulose, diluents, lubricants, binders and disintegrating agents. If desired, tablet dosages of the disclosed compositions may be coated by standard aqueous or nonaqueous techniques, "Pharmaceutically acceptable carrier" also encompasses controlled release means.

Compositions of the present invention may also optionally include other therapeutic ingredients, anti-caking agents, preservatives, sweetening agents, colorants, flavors, desiccants, plasticizers and dyes. Any such optional ingredient must, of course, be compatible with the compound of the invention to insure the stability of the formulation.

For topical application, there are employed as non-sprayable forms, viscous to semi-solid or solid forms comprising a carrier compatible with topical application and having a dynamic viscosity preferably greater than water. Suitable formulations include but are not limited to solutions, suspensions, emulsions, creams, ointments, powders, liniments, salves, aerosols, etc., which are, if desired, sterilized or mixed with auxiliary agents, e.g., preservatives, stabilizers, wetting agents, buffers or salts for influencing osmotic pressure, etc. For topical application, also suitable are sprayable aerosol preparations wherein the active ingredient, preferably in combination with a solid or liquid inert carrier material, is packaged in a squeeze bottle or in admixture with a pressurized volatile, normally gaseous propellant, e.g., a freon.

The topical pharmaceutical carrier may include any substance capable of dispersing and maintaining contact between the active ingredients and the skin. The vehicle may be glycerin, alcohol or water based. Examples of such vehicles include aloe vera, which is a gel base, together with ethanol, isopropyl alcohol, water, propylene glycol and a non-ionic surfactant such as laureth-4. Other water-based alcohol/ glycerin vehicles and carriers are within the scope of the present invention. A typical water-based lotion will contain from 45 to 50 parts of glycerin, one to three parts Tween 80TM, from 45 to 50 parts of water and from 1 to 50 parts of the compound of the invention.

Also included in the scope of the invention are ointments, emulsions or dispersions in which water, if present, is a minor constituent. Typical ointment formulation comprises from 90 to 98 parts of a mixture of petrolatum, mineral oil, mineral wax and wool wax alcohol, from 0.5 to 3 parts of a mixture of polyoxyethylene and sorbitan monooleate (Tween 80TM), from 1 to 5 parts of water, and from 1 to 50 parts of the compound of the invention. Another suitable non-aqueous ointment can be prepared from 95 parts of liquid petrolatum USP, 5 parts polyethylene and from 1 to 50 parts of the compound of the invention. The resulting ointment spreads easily and has an even consistency over wide temperature extremes. It is, in addition, non-irritating and non-sensitizing.

Formulations of the compounds of the invention may also be prepared containing from 0 to 25% by weight of urea. In general, in such urea containing ointments, the water content will vary from 5 to 50% by weight of the composition. Any suitable ointment carrier may be used such as lanolin and ethylene glycol polymers. In the case of formulations containing urea, it is known in the art that borate salts may often be added to stabilize the pharmaceutical composition (see U.S. patent 2,917,433, the disclosure of which is incorporated herein by reference).

Water based compositions may also be employed, in which case the compound of the invention will commonly be in solution, and the aqueous solution may, if desired, be thickened with a suitable gel to provide a less mobile composition. Such compositions are well known in the art

### Experimental Procedures and Results

Condensing agents for reacting amines (e.g. spermine)with carboxylic acids (e.g. cholic acid) are well known, particularly in the art of synthesis of peptides. Such agents include carbodiimides of various sorts, mixed anhydrides, EEDQ, HATU. It is also possible to pre-react the carboxylic acid of the linker with an appropriate leaving group to form an activated ester. Activated esters denote esters which are capable of undergoing a substitution reaction with primary or secondary amines to form an amide. The term includes esters "activated" by neighboring electron withdrawing substituents. Examples include esters of phenols, particularly electronegatively substituted phenol esters such as pentafluorophenol esters; O-esters of isourea, such as arise from interaction with carbodiimides; O-esters of N-hydroxyimides and N-hydroxy heterocycles; specific examples include S-t-butyl esters, S-phenyl esters, S-2-pyridyl esters, N-hydroxypiperidine esters, N-hydroxysuccinimide esters, N-hydroxyphthalimide esters and N-hydroxybenzotriazole esters.

**Lysine-dicholamide**. The procedure used for the synthesis of lysine-dicholamide was similar to that described in the literature. Thus, to a solution of cholic acid (6.08 g, 14.9 mmol) and *N*-hydroxysuccinimide (1.90 g, 16.5 mmol) in anhydrous THF (50 mL) was added DCC (3.60 g, 17.4 mmol). The resulting reaction mixture was stirred at room temperature. After 4 hrs, the reaction mixture was filtered to remove insoluble urea. The filtrate was then dropped into a solution of *L-*lysine (950 mg, 6.50 mmol) and triethylamine (6.2 mL) in H₂O (20 mL). The reaction mixture was stirred overnight. After concentrating until some precipitates appeared, the reaction mixture was poured into 1 M hydrochloric acid (200 mL). The resulting solid was collected by filtration. The residue was purified by chromatography on a silica-gel column, eluted with CHCl₃/MeOH/H₂O (40/10/1 by volume) to afford lysine-dicholamide (4.51 g, 75%). Synthesis of

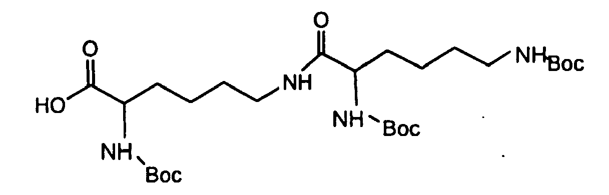

A solution of Boc-*L*-Lys-OH (200 mg, 0.812 mmol) and Boc-*L*-Lys(Boc)-OSu (317 mg, 0.834 mmol) in THF-H₂O (16.5 mL, 10/1) was stirred at room temperature for 2 h. The reaction mixture was concentrated under vacuum. The obtained residues were partitioned between water and chloroform. The organic layer was separated and the aqueous layer was extracted with chloroform thrice. The combined organic layers were washed with water and dried over anhydrous sodium sulfate. Purification was accomplished by chromatography on a silica-gel column, eluted with CHCl₃/MeOH (40/1, v/v) to afford 369 mg (79%) of product. ¹H NMR (500 MHz, CD₃OD, 298 K) δ 4.01 (br, 1H), 3.93 (br, 1 H), 3.20 (m, 2H), 3.02 (t, ³*J* = 6.78 and 6.91 Hz, 2 H), 1.79-1.30 (m, 39 H).
To a solution of lysine-dicholamide (120 mg, 0.129 mmol) in 1 mL of DMF were added dihydro-4-oxo-1,2,3-benzotriazine (DHBT) (25 mg, 0.153 mmol) and DCC (32 mg, 0.155 mmol). After stirring for the mixture for 3 h, spermine (5.5 mg, 0.027 mmol) and triethylamine (54 mL) were added. The reaction mixture was stirred overnight, and then poured into 50 mL of 1 M aqueous HCl. The resulting precipitate was collected by filtration, and purified by preparative thin layer chromatography [silica, CHCl₃/CH₃OH/H₂O (40/10/1, v/v/v)] to give 79 mg (76%) of the desired conjugate (**5b**) spermine/lysine₄/Chol₈ having R_{f} 0.43. [silica, CHCl₃/CH₃OH/H₂O (40/10/1, v/v/v)] and ¹H NMR (CD₃OD, 500 MHz, 318 K) d ppm: 0.70 (s, 24 H), 0.90-2.31 (m, 272 H), 3.16 (m, 12 H), 3.33-3.50 (m, 16 H), 3.79 (s, 8 H), 3.94 (s, 8 H), 4.25 (br, 2 H), 4.75 (br, 2 H). HRMS for C226H378N12O36Na+ Calcd: 3859.8009. Found: 3859.8264.

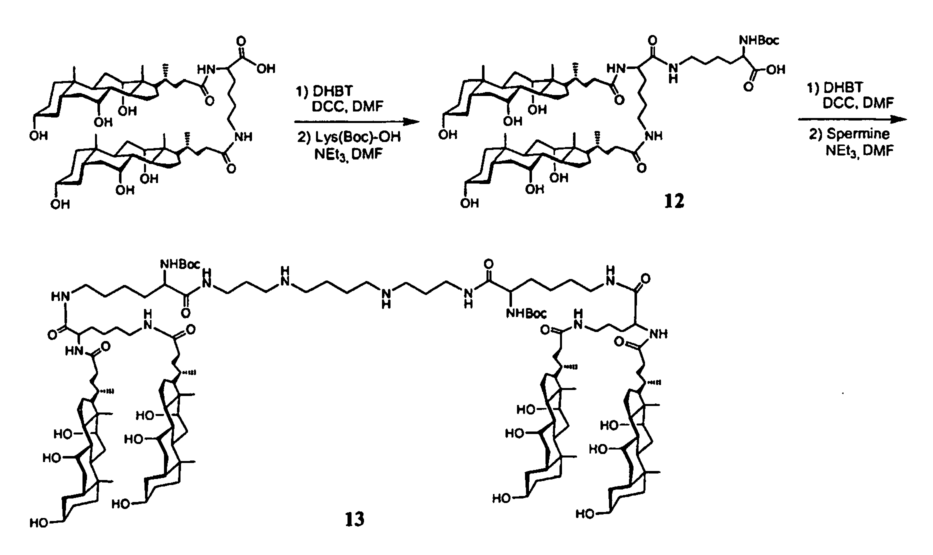

To a solution of lysine-dicholamide (3.7 g, 4 mmol) in 10mL of DMF was added dihydro-4-oxo-1,2,3-benzotriazine (DHBT) (1 g, 6.4 mmol, 1.6eq) and DCC (825 mg, 4 mmol, 1eq). After stirring the mixture for 3h at room temperature, Lys(Boc)-OH (790 mg, 3.2 mmol, 0.8 eq) and triethlyamine(0.2 mL) were added. The reaction mixture was stirred overnight at room temperature and then poured into 50 mL of diluted aqueous HCl. The resulting precipitate was collected by filtration and purified by column chromatography (silica, CHCl₃/CH₃OH/H₂O: 65/25/4, *Rƒ* = 0.43) to give 2.51 g (yield: 68%) of the desired product **12**. ¹H NMR (CD₃OD, 500MHz) δ 0.696 (s, 6H), 0.909-1.022 (m, 16H), 1.383-2.291 (m, 75H), 3.142-3.155 (m, 4H), 3.307 (m, 2H), 3.784 (s, 2H), 3.941 (s, 2H), 3.996 (br, 1H), 4.245 (m, 1H). MALDI-TOF MS *mlz* 1178 ([M+Na]⁺).

Synthesis of **13** To a solution of 12 (302 mg, 0.26mmol) in 5mL of DMF was added DHBT (100 mg, 0.64 mmol, 2.5 eq) and DCC (100 mg, 0.5 mmol, 2eq). After stirring the mixture for 3h at room temperature, spermine (26 mg, 0.13 mmol, 0.25 eq) and triethlyamine (0.1 mL) were added. The reaction mixture was stirred overnight at room temperature and then poured into 50 mL of diluted aqueous HCl. The resulting precipitate was collected by filtration and purified by column chromatography (silica, CHCl₃/CH₃OH/H₂O: 65/25/4, *Rƒ*=0.26) to give 212mg (yield: 66%) of the desired product **13**. ¹H NMR (CD₃OD, 500MHz) δ 0.710 (s, 12H), 0.920-1.095 (m, 32H), 1.405-1.970 (m, 166H), 2.604 (br, 8H), 3.158 (br, 8H), 3.167-3.349 (m, 4H), 3.795 (s, 4H), 3.952 (br, 6H), 4.227 (br, 2H). MALDI-TOF MS *m*/*z* 2478 ([M+H]⁺).

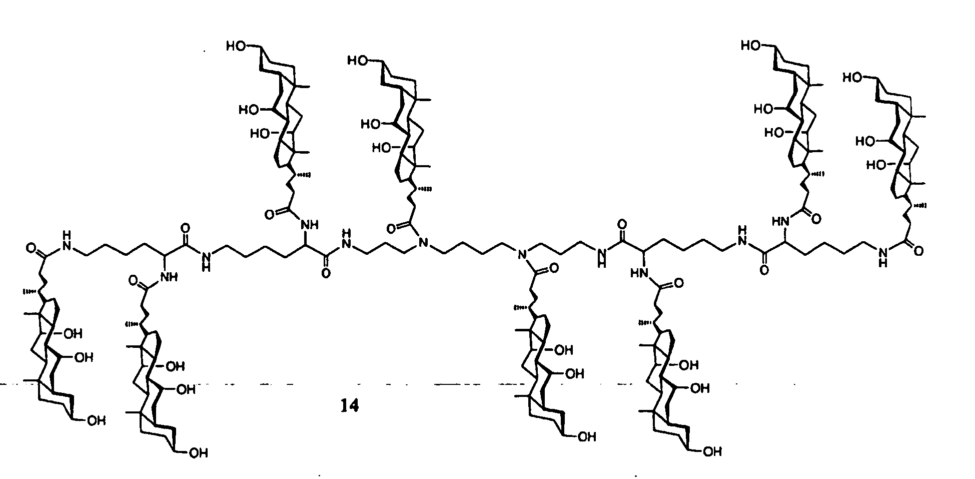

Synthesis of conjugate **14** The solution of **13** (200 mg, 0.08 mmol) in 5 mL of chloroform and TFA (1:1) was stirred for 4hrs at room temperature. After evaporated the solution, the gotten oil was used directly for next step reaction. To a solution ofcholic acid (410 mg, 1 mmol) in 5 mL of DMF was added DHBT (200 mg, 1.2 mmol) and DCC (240 mg, 1.2 mmol). After stirring the mixture for 3hrs at room temperature, the gotten oil and triethylamine (0.1 mL) were added. The reaction mixture was stirred overnight at room temperature and then poured into 50 mL of diluted aqueous HCl. The resulting precipitate was collected by filtration and purified by column chromatography (silica, CHCl₃/CH₃OH/H₂O: 65/25/4, *Rf*= 0.52) to give 112 mg (yield: 36%) of the desired product **14**. ¹H NMR (CD₃OD, 500 MHz) δ 0.715 (s, 24 H), 0.919-1.045 (m, 64 H), 1.396-2.296 (m, 242 H), 3.178 (br, 12 H), 3.304-3.349 (br, 16 H), 3.805 (s, 8 H), 3.951 (br, 8 H), 4.259 (br, 4 H). MALDI-TOF MS *m*/*z* 3862 ([M+Na]⁺).

Sulfation of spermine/lysine₄/Chol₈ (**5b**) was carried out by dissolving 147 mg (0.038 mmol) of spermine/lysine₄/Chol₈ (**5b**) in 5 mL of DMF at 0°C, followed by direct addition of 435 mg (2.73 mmol) of sulfur trioxide/pyridine complex. After stirring for 10 h at room temperature, 2 mL of cold water was added, followed by addition of saturated sodium bicarbonate until the pH of the mixture was 10. The combined solvent was then removed under reduced pressure, and the residue purified by column chromatography [silica, CHCl₃/CH₃OH/H₂O(4/4/1, v/v/v)] to give 188 mg (78%) of spermine/lysine₄/CTS₈ **5a** as its sodium salt having Rf 0.45 and ¹H NMR (CD₃OD, 45° C): 0.76(s, 24 H), 0.92-2.43(m, 272 H), 3.15(m, 12 H), 3.3(m, 2 H), 3.43(m, 6 H), 4.14(m, 8 H), 4.26(m, 2 H), 4.45(s, 8 H), 4.66(s, 8 H), 4.76(m, 2 H).

Sulfation of spermidinelChol₃ was carried out in similar fashion to that described above, to give a 75% yield of spermidine/CTS₃ as its sodium salt having Rf 0.34 [silica, CHCl₃/CH₃OH/H₂O (50/40/10, v/v/v)] and ¹HNMR (CD₃OD, rt): 0.76(s, 9H), 0.93-2.40(m, 96H); 3.13-3.20(m, 4 H), 3.28-3.36(m, 4H), 4.11(m, 3 H), 4.44(s, 3 H), 4.66(s, 3 H).

Sulfation of putrescine/lysine₂/Chol₄ was carried out in similar fashion to that described above, to give a 81% yield of putrescine/lysine₂/CTS₄ as its sodium salt having Rf 0.32 [silica, CHCl₃/CH₃OH/H₂O (50/40/10, v/v/v)] and ¹HNMR (CD₃OD, rt): ¹HNMR (CD₃OD, rt): 0.76(s, 12 H), 0.92-2.43(m, 136 H), 3.14(m, 8 H), 4.12(m, 4 H), 4.20(m, 2 H), 4.44(s, 4 H), 4.65(s, 4 H).

Sulfation of spermidine/lysine₃/Chol₆ was carried out in similar fashion to that described above, to give a 64% yield of spermidine/lysine₃/CTS₆ as its sodium salt having Rf 0.47 [silica, CHCl₃/CH₃OH/H₂O (40/40/10, v/v/v)] and ¹HNMR (CD₃OD, rt): ¹HNMR (CD₃OD, 45°C): 0.76(s, 18 H), 0.91-2.43(m, 204 H), 3.15(m, 10 H), 3.3(m, 2 H), 3.43(m, 2 H), 4.14(m, 6 H), 4.26(m, 2 H), 4.45(s, 6 H), 4.66(s, 6 H).

The anti-HIV activity of these conjugates was measured using U87.CD4.CCR5 cells, replication defective HIV-1 virus and a luciferase assay as described by Herold *et al.* J Virol. 2002, 76, 11236. At a dosage of 1µg/mL, the spermine/lysine₄/CTS₈ derivative 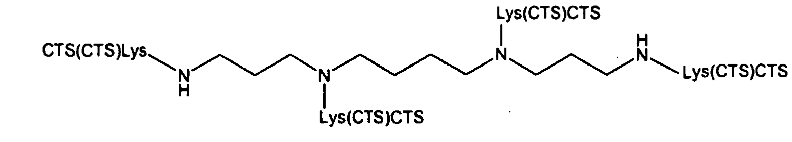 in which CPS is cholic acid trisulfate (CTS) showed moderate activity. The corresponding putrescine derivative, putrescine/lysine₂/CTS₄, and spermidine derivative, spermidine/lysine₃/CTS₆ , were less active. A compound having only 3 cholanetrisulfate residues, spermidine/CTS₃, was inactive. The anti-HSV activity was also compared by conducting plaque reduction assays with HSV-2(G) and human cervical epithelial cells (CaSki). At a concentration of 10 µg/mL, complete and very significant inhibition was found for spermine/lysine₄/CTS₈ and spermidine/lysine₃/CTS₆. In contrast, the activities of spermidine/CTS₃, putrescine/lysine₂/CTS₄ and spermine/CTS₄ were only modest at this concentration.

## Claims

1. A compound of formula 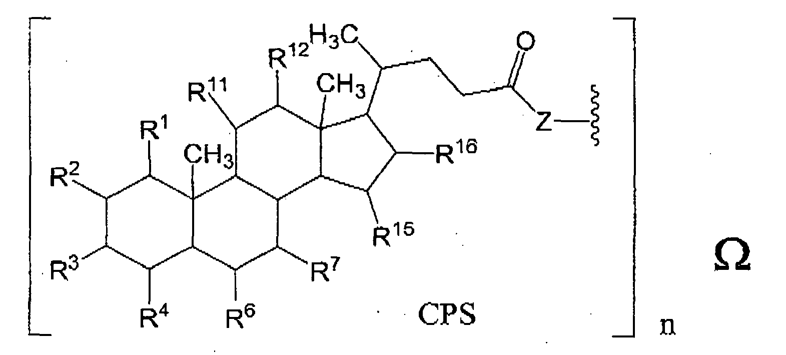 wherein
CPS is a cholanic acid derivative having an α face and a β face;
R¹ to R¹⁶ are chosen independently from H, OH and OSO₃⁻A⁺, with the proviso that at least two of R¹ to R¹⁶ are -W-OSO₃⁻A^{÷} and are on the same face of CPS;
W is a direct bond or a deshydrogen residue of a C₃-C₆ diol or triol;
A is chosen from H⁺, a quaternary ammonium species and the cation of an alkali or alkaline earth metal;
Z is a direct bond or a residue of an amino acid, a peptide or an aminosulfonic acid;
Ω is a moiety having n or more -NH-, 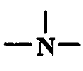 and -O- groups separated by 6 or fewer carbons, to which -NH-, 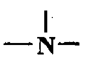 and -O- groups said CPS is attached;
and
n is an integer from 6 to 200.

2. A compound according to claim 1 wherein n is an integer from 8 to 24.

3. A compound according to claim 1 wherein Ω is a dendritic oligomer.

4. A compound according to claim 1 wherein Ω is a linear Oligomer.

5. A compound according to claim 1 wherein Ω is a poly(vinyl alcohol) or poly (ethyleneimine).

6. A compound according to claim 1 wherein Ω is a spermine/lysine backbone.

7. A compound according to claim 6 wherein Q is a spermine/lysine₄ backbone.

8. A compound according to claim 7 wherein Ω is 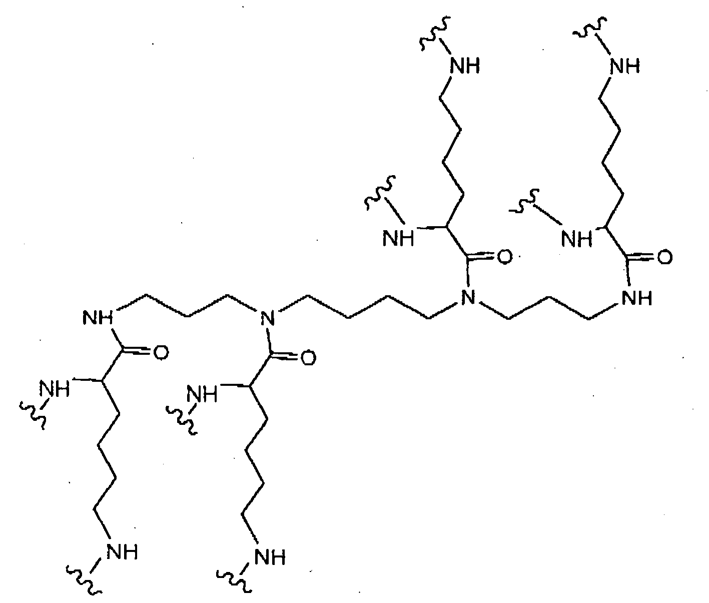 and the wavy lines indicate points of attachment of CPS residues.

9. A compound according to claim 7 wherein Ω is 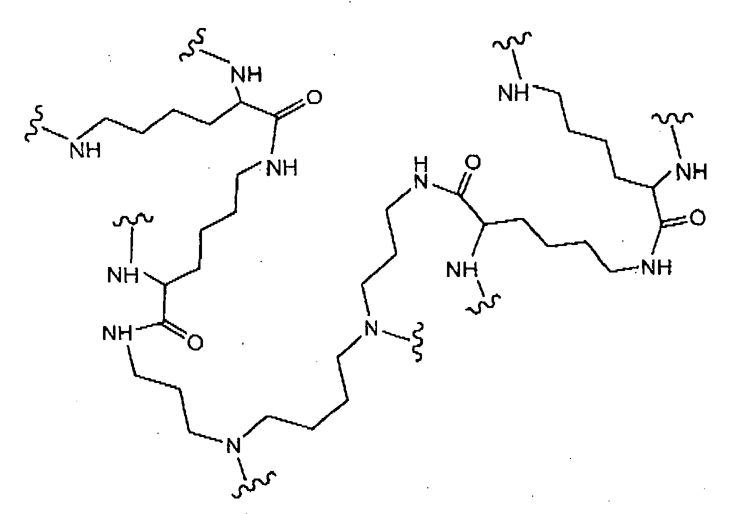 and the wavy lines indicate points of attachment of CPS residues.

10. A compound according to claim 1 wherein at least two of R³, R⁶, R⁷ and R¹² are OSO₃⁻A^{÷} and the remainder of R¹ to R¹⁶ are H.

11. A compound according to any of claims 1 to 10 wherein Z is a direct bond and CPS is:

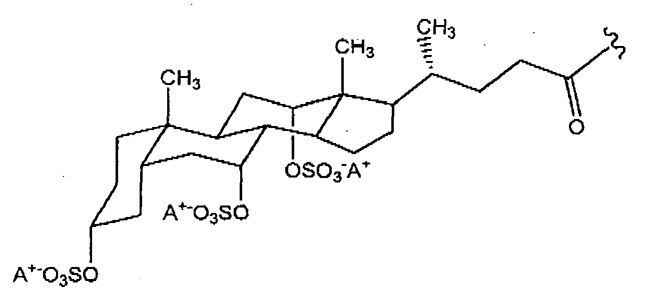

12. A compound according to any of claims 1 to 10 wherein CPS is:

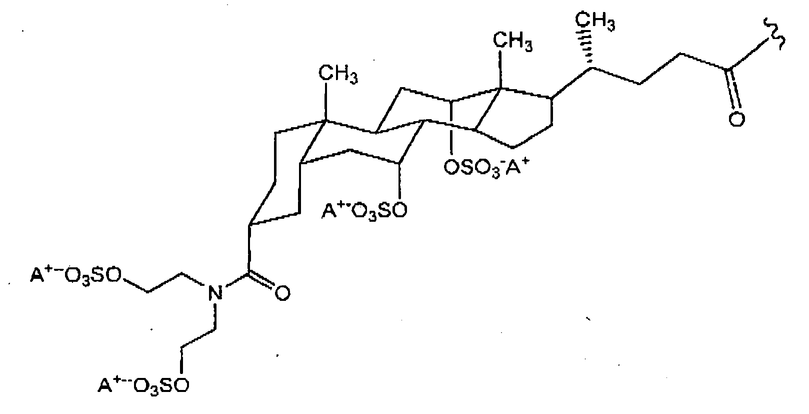

13. A compound according to any of claims 1 to 10 wherein CPS is chosen from: 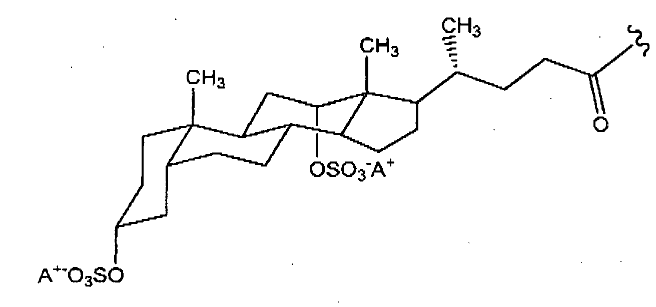 and

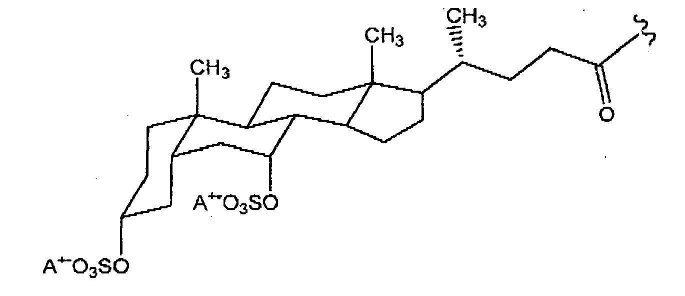

14. A compound according to any one of claims 1 to 13 for use in inhibiting the infectivity of a virus.

15. A compound according to claim 14 wherein the virus is a human immunodeficiency virus or a herpes simplex virus.

16. Use of a compound according to any one of claims 1 to 13 for the manufacture of a medicament for use in inhibiting the infectivity of a virus.

17. Use according to claim 16 wherein the virus is a human immunodeficiency virus or a herpes simplex virus.

18. A pharmaceutical composition comprising a pharmaceutically acceptable carrier and a compound according to any one of claims 1 to 13.

19. A pharmaceutical composition according to claim 18 wherein said carrier is a carrier for topical application.

## Patentansprüche

1. Verbindung mit der Formel 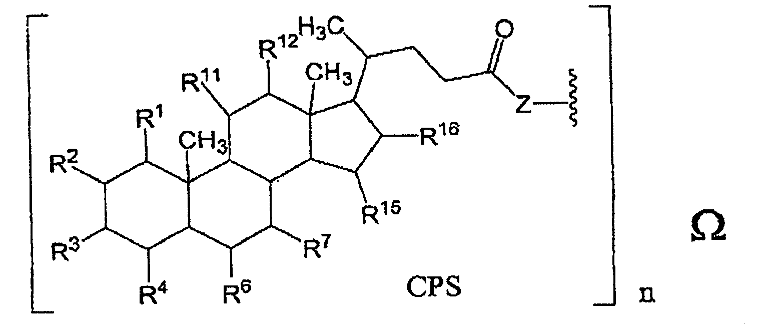 wobei
CPS ein Cholansäurederivat mit einer α-Fläche und einer β-Fläche ist;
R¹ bis R¹⁶ unabhängig voneinander unter H, OH und OSO₃⁻A⁺ ausgewählt werden, unter dem Vorbehalt, daß mindestens zwei der Komponenten R¹ bis R¹⁶ -W-OSO₃⁻A⁺ sind und sich auf der gleichen CPS-Fläche befinden;
W eine Direktbindung oder ein wasserstofffreier Rest eines C₃-C₆-Diols oder -Triols ist;
A unter H⁺, einer quaternären Ammonium-Spezies und dem Kation eines Alkali- oder Erdalkalimetalls ausgewählt ist;
Z eine Direktbindung oder ein Aminosäure-, Peptid- oder Aminosulfonsäurerest ist;
Ω eine Komponente mit n oder mehr -NH-, 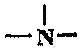 und -O- -Gruppen ist, die durch 6 oder weniger Kohlenstoffatome getrennt sind, an welche -NH-, 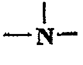 und -O- -Gruppen das CPS gebunden ist;
und
n eine ganze Zahl von 6 bis 200 ist.

2. Verbindung nach Anspruch 1, wobei n eine ganze Zahl von 8 bis 24 ist.

3. Verbindung nach Anspruch 1, wobei Ω ein dendritisches Oligomer ist.

4. Verbindung nach Anspruch 1, wobei Ω ein lineares Oligomer ist.

5. Verbindung nach Anspruch 1, wobei Ω ein Poly(vinylalkohol) oder Poly(ethylenimin) ist.

6. Verbindung nach Anspruch 1, wobei Ω eine Spermin/Lysin-Hauptkette ist.

7. Verbindung nach Anspruch 6, wobei Ω eine Spermin/Lysin₄-Hauptkette ist.

8. Verbindung nach Anspruch 7, wobei Ω durch 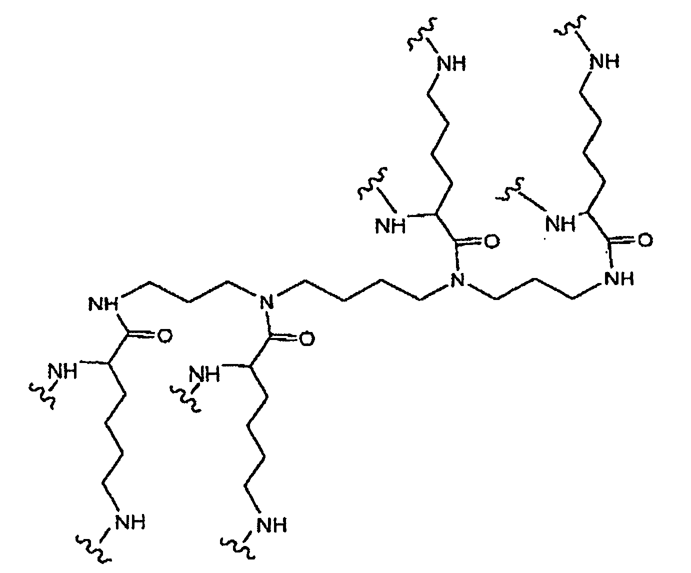 gegeben ist und die Schlangenlinien Bindungsstellen von CPS-Resten andeuten.

9. Verbindung nach Anspruch 7, wobei Ω durch 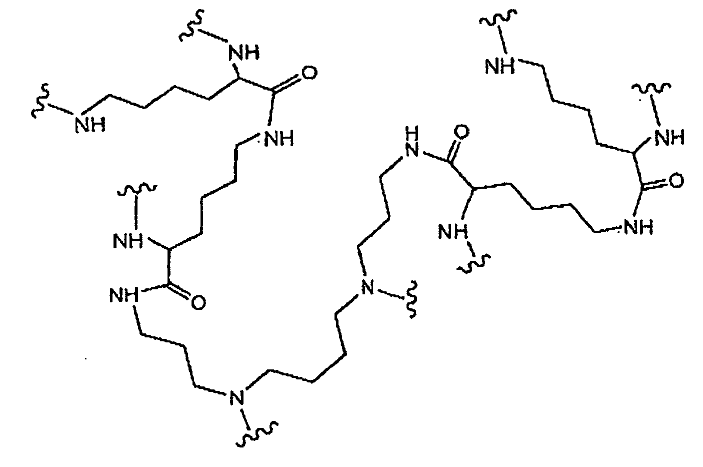 gegeben ist und die Schlangenlinien Bindungsstellen von CPS-Resten andeuten.

10. Verbindung nach Anspruch 1, wobei mindestens zwei der Komponenten R³, R⁶, R⁷ und R¹² für OSO₃⁻A⁺ stehen und die übrigen von den Komponenten R¹ bis R¹⁶ für H stehen.

11. Verbindung nach einem der Ansprüche 1 bis 10, wobei Z eine Direktbindung ist und CPS durch 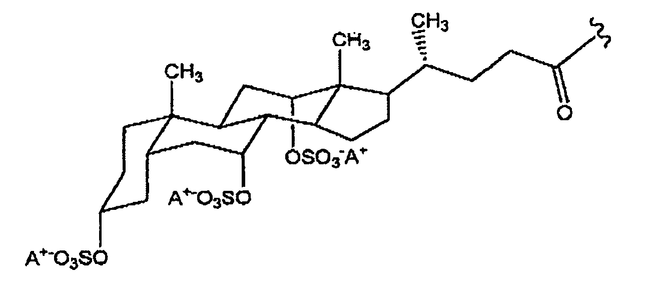 gegeben ist.

12. Verbindung nach einem der Ansprüche 1 bis 10, wobei CPS durch 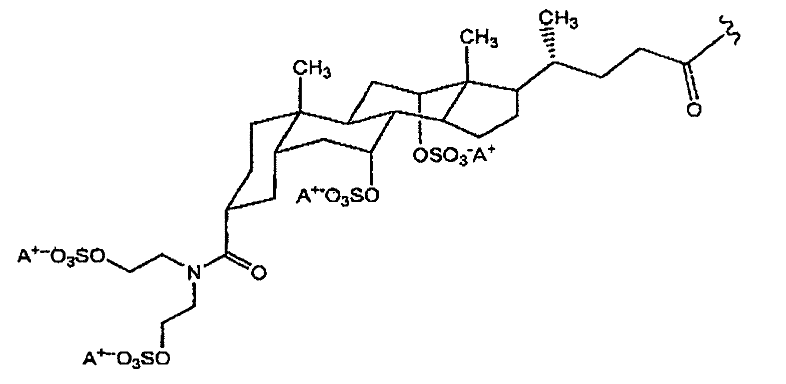 gegeben ist.

13. Verbindung nach einem der Ansprüche 1 bis 10, wobei CPS unter 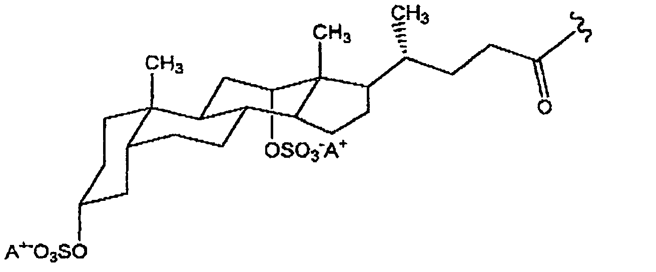 und 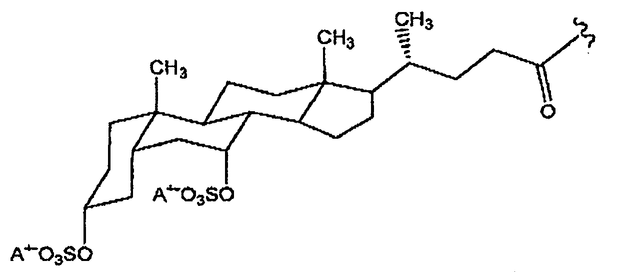 ausgewählt ist.

14. Verbindung nach einem der Ansprüche 1 bis 13 zur Verwendung bei der Hemmung der Infektiosität eines Virus.

15. Verbindung nach Anspruch 14, wobei das Virus ein HIV-Virus oder ein Herpes-simplex-Virus ist.

16. Verwendung einer Verbindung nach einem der Ansprüche 1 bis 13 für die Herstellung eines Medikaments zur Anwendung bei der Hemmung der Infektiosität eines Virus.

17. Verwendung nach Anspruch 16, wobei das Virus ein HIV-Virus oder Herpes-simplex-Virus ist.

18. Pharmazeutische Zusammensetzung, die einen pharmazeutisch akzeptierbaren Träger und eine Verbindung nach einem der Ansprüche 1 bis 13 aufweist.

19. Pharmazeutische Zusammensetzung nach Anspruch 18, wobei der Träger ein Träger für topische Anwendung ist.

## Revendications

1. Composé suivant la formule : 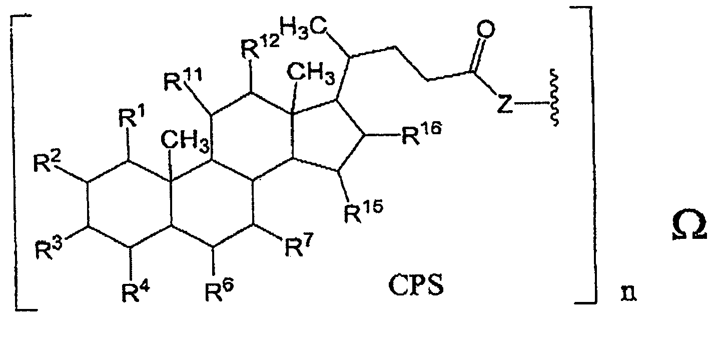 dans lequel ;
CPS est un dérivé de l'acide cholanique possédant une face α et une face β;
R¹ à R¹⁶ sont choisis indépendamment parmi H, OH et OSO3⁻A⁺, avec la restriction qu'au moins deux entre les R¹ à R¹⁶ soient constituées de -W-OSO₃⁻A⁺ et que ceux-ci se trouvent sur la même face du CPS;
W est constitué par une liaison directe ou par un résidu déshydrogène d'un diol ou triol C₃-C₆ ;
A est choisi parmi H⁺, une espèce d'ammonium quaternaire et le cation d'un alcali ou d'un métal alcalino-terreux ;
Z est constitué par une liaison directe ou par un résidu d'un acide aminé, d'un peptide ou d'un acide aminosulfonique ;
Ω est une moitié comportant n ou davantage de groupes -NH-, 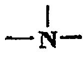 et -O- séparés par 6 carbones ou moins, à laquelle les groupes -NH-, 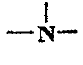 et -O- dudit CPS sont rattachés ;
et
n est un nombre entier compris entre 6 et 200.

2. Composé suivant la revendication 1 dans lequel n est un nombre entier compris entre 8 et 24.

3. Composé suivant la revendication 1 dans lequel Ω est un oligomère dendritique.

4. Composé suivant la revendication 1 dans lequel Ω est un oligomère linéaire.

5. Composé suivant la revendication 1 dans lequel Ω est un alcool polyvinylique ou une imine polyéthylénique.

6. Composé suivant la revendication 1 dans lequel Ω est une chaîne principale de spermine/lysine.

7. Composé suivant la revendication 6 dans lequel Ω est une chaîne principale de spermine/lysine₄.

8. Composé suivant la revendication 7 dans lequel Ω est ; 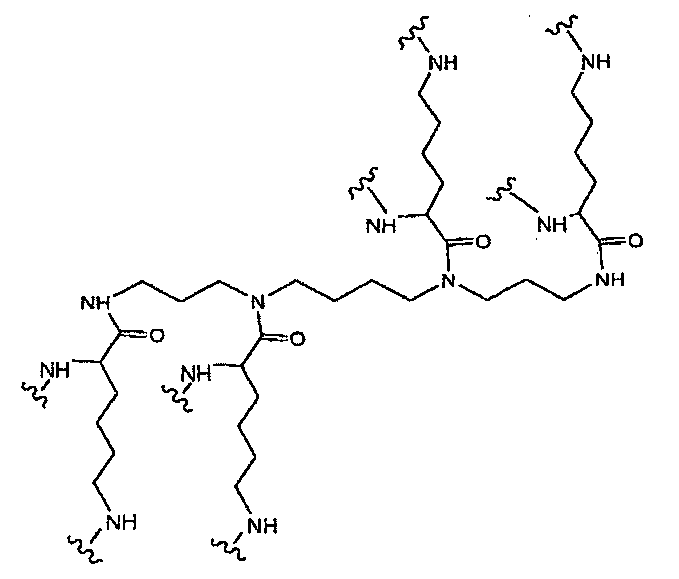 et les lignes ondulées indiquent des points de rattachement de résidus CPS.

9. Composé suivant la revendication 7 dans lequel Ω est ; 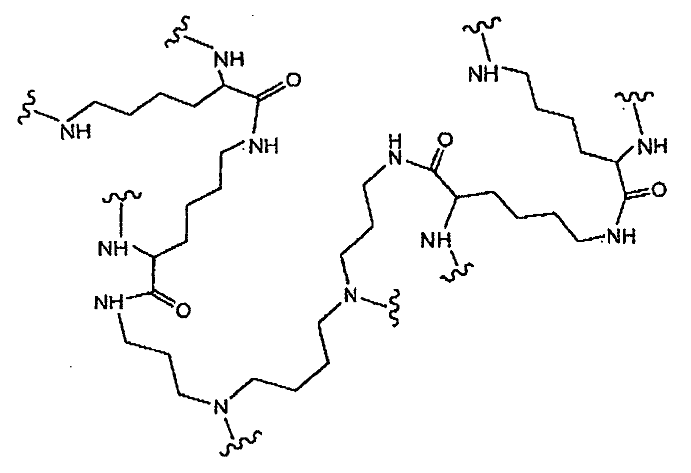 et les lignes ondulées indiquent des points de rattachement de résidus CPS.

10. Composé suivant la revendication 1 dans lequel au moins deux parmi R³, R⁶, R⁷ et R¹² sont constitués de OSO₃⁻A⁺ et le reste des R¹ à R¹⁶ est constitué de H.

11. Composé suivant une quelconque des revendications 1 à 10 dans lequel Z est une liaison directe et CPS est ;

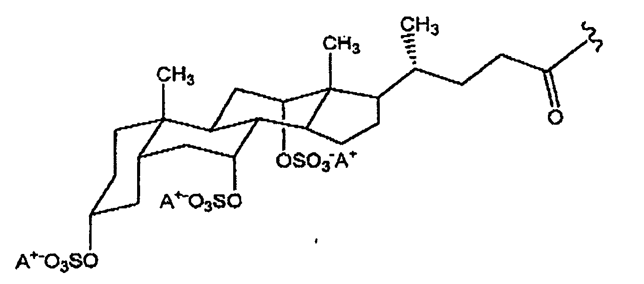

12. Composé suivant une quelconque des revendications 1 à 10 dans lequel CPS est ;

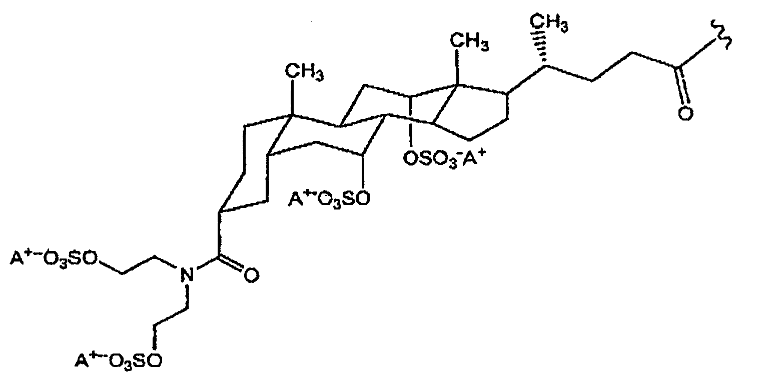

13. Composé suivant une quelconque des revendications 1 à 10 dans lequel CPS est choisi parmi : 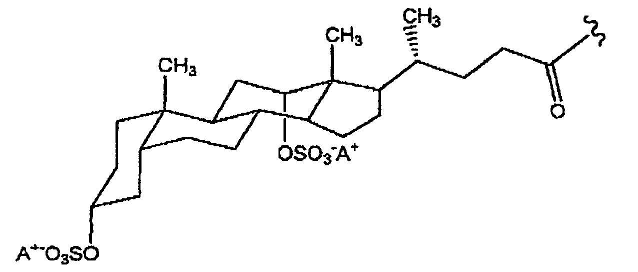 et

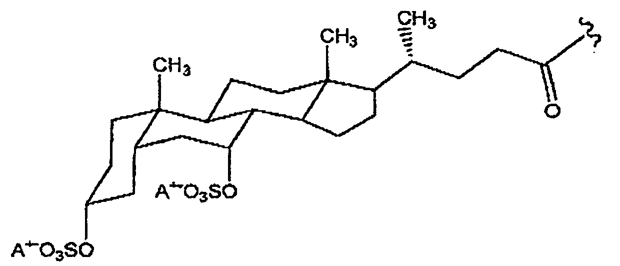

14. Composé suivant une quelconque des revendications 1 à 13 pour la mise en oeuvre en vue de l'inhibition de la contagiosité d'un virus.

15. Composé suivant la revendication 14 dans lequel le virus est un virus de l'immunodéficience humaine ou un virus herpès simplex.

16. Utilisation d'un composé suivant une quelconque des revendications 1 à 13 pour la fabrication d'un médicament pour la mise en oeuvre en vue de l'inhibition de la contagiosité d'un virus.

17. Utilisation suivant la revendication 16 dans laquelle lequel le virus est un virus de l'immunodéficience humaine ou un virus herpès simplex.

18. Composition pharmaceutique comportant un support pharmaceutiquement acceptable ainsi qu'un composé suivant une quelconque des revendications 1 à 13.

19. Composition pharmaceutique suivant la revendication 18 dans laquelle le support est un support pour une application topique.
